(19) Europäisches Patentamt
European Patent Office
Office européen des brevets

(11) Veröffentlichungsnummer: **0 198 351**
**B1**

(12) # EUROPÄISCHE PATENTSCHRIFT

(45) **Veröffentlichungstag der Patentschrift: 18.10.89**

(21) **Anmeldenummer: 86104586.2**

(22) **Anmeldetag: 04.04.86**

(51) Int. Cl.⁴: **C 07 B 41/00**, C 07 J 9/00, C 07 C 49/613, C 07 C 45/34, C 07 C 33/02, C 07 C 29/50, C 07 C 179/02, C 07 C 178/00

(54) **Katalytische Oxidation mit N-Hydroxydicarbonsäureimid.**

(30) Priorität: **17.04.85 CH 1637/85**

(43) Veröffentlichungstag der Anmeldung:
**22.10.86 Patentblatt 86/43**

(45) Bekanntmachung des Hinweises auf die Patenterteilung:
**18.10.89 Patentblatt 89/42**

(84) Benannte Vertragsstaaten:
**AT BE CH DE FR GB IT LI NL**

(56) Entgegenhaltungen:
**EP-A- 0 067 258**
**US-A- 2 911 442**

**CHEMICAL ABSTRACTS, Band 84, Nr. 23, 7. Juni 1976, Seite 423, Zusammenfassung Nr. 164279h, Columbus, Ohio, US**

(73) Patentinhaber: **F. HOFFMANN-LA ROCHE AG, Postfach 3255, CH-4002 Basel (CH)**

(72) Erfinder: **Foricher, Joseph, 34 Rue Daguerre, F-68200 Mulhouse (FR)**
Erfinder: **Fürbringer, Claude, 80 Unterm Schellenberg, CH-4125 Riehen (CH)**
Erfinder: **Pfoertner, Karlheinz, Dr., 24 Gellertstrasse, CH-4052 Basel (CH)**

(74) Vertreter: **Zimmermann, Hans, Dr. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein Verfahren zur katalytischen Oxidation von allylischen Gruppen. Derartige Reaktionen sind in der organischen Chemie grundsätzlich von großem Interesse und finden in verschiedensten Bereichen Anwendung, wie beispielsweise bei der Herstellung von Steroiden, Vitaminen, Riechstoffen, Carotinoiden und dergleichen.

Zur Oxidation allylischer Gruppen wurden bisher häufig Metalloxide, beispielsweise Mangandioxid, Chromsäure oder Chromate verwendet. Ferner sind katalytische Oxidationen mit Sauerstoff oder Luft bekannt, wobei als Katalysatoren Metallverbindungen, beispielsweise Cobalt-, Mangan-, Lanthan- oder Rhodiumverbindungen, eingesetzt wurden (siehe z.B. EP-A-0067258).

Die vorbekannten Verfahren ergaben jedoch häufig unbefriedigende Ausbeuten und führen zu Umweltbelastung durch die anfallenden Metallsalze. Zudem enthalten die Produkte meist noch Spuren von Metallverbindungen, was insbesondere bei der Herstellung von Pharmazeutika, Nahrungs- und Futtermittelzusätzen unerwünscht ist.

Die vorliegende Erfindung betrifft nun ein neues Verfahren zur katalytischen Oxidation von Isoprenoiden, welche eine allylische Gruppe besitzen, mit Sauerstoff oder einem sauerstoffhaltigen Gas. Das erfindungsgemäße Verfahren ist dadurch gekennzeichnet, daß man ein Isoprenoid, welches mindestens ein allylisches Wasserstoffatom aufweist, in einem inerten Keton oder Ester in Gegenwart eines N-Hydroxydicarbonsäureimids der allgemeinen Formel

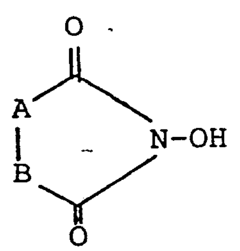 I

worin A–B für $CH_2$-$CH_2$, CH=CH, einen aromatischen Kohlenwasserstoffrest oder eine von diesen Gruppen abgeleitete Gruppe steht, worin ein oder mehrere Wasserstoffatome durch Alkyl oder Halogen ersetzt sind, oxidiert, und daß man, gewünschtenfalls, ein erhaltenes Hydroperoxid zum Alkohol reduziert oder ein erhaltenes primäres oder sekundäres Hydroperoxid zur Carbonylverbindung dehydratisiert.

Das erfindungsgemäße Verfahren vermeidet die Verwendung von Metallverbindungen und somit auch eine Verunreinigung des Produktes durch Metallverbindungen. Die erfindungsgemäß verwendeten Katalysatoren der Formel I sind vergleichsweise toxikologisch unbedenklich. Da zudem der Katalysator durch einfache Maßnahmen praktisch vollständig zurückgewonnen und wiederverwendet werden kann, wird auch die Umweltbelastung erheblich vermindert. Das erfindungsgemäße Verfahren ist zudem technisch leicht anwendbar und billig, da es insbesondere auch mit Luft durchgeführt werden kann.

Der Ausdruck «Alkyl» umfaßt im Rahmen der vorliegenden Erfindung geradkettige und verzweigte Alkylgruppen, wie Methyl, Äthyl, Propyl, Isopropyl, Butyl, Isobutyl, t-Butyl und dergleichen.

Der Ausdruck «Halogen» bedeutet im Rahmen der vorliegenden Erfindung Fluor, Chlor, Brom oder Jod, vorzugsweise Chlor.

Der Ausdruck «primäres Hydroperoxid» bezeichnet Verbindungen, welche eine Hydroperoxymethyl-Gruppe $-CH_2-OOH$ aufweisen, und der Ausdruck «sekundäres Hydroperoxid» bezeichnet Verbindungen, welche eine Hydroperoxymethylen-Gruppe -CH(OOH)- aufweisen.

Die erfindungsgemäß verwendeten Katalysatoren sind N-Hydroxyimide von Dicarbonsäuren, wobei selbstverständlich nur solche Dicarbonsäuren in Betracht kommen, welche cyclische Imide gemäß Formel I bilden können. Der Ausdruck «aromatischer Kohlenwasserstoffrest» in obiger Formel I umfaßt somit von aromatischen Kohlenwasserstoffen, wie Benzol, Naphthalin und dergleichen, abgeleitete Diradikale, wobei die Radikalstellen in ortho- oder peri-Stellung zueinander stehen, beispielsweise 1,2-Phenylen, 1,2-Naphthylen, 2,3-Naphthylen und 1,8-Naphthylen. Bevorzugte Alkylsubstituenten in Formel I sind diejenigen mit 1–4 Kohlenstoffatomen und bevorzugter Halogensubstituent ist Chlor.

Beispiele bevorzugter Katalysatoren der Formel I sind N-Hydroxysuccinimid, N-Hydroxymaleinsäureimid, N-Hydroxyphthalimid, N-Hydroxy-2,3-naphthalindicarbonsäureimid und die davon abgeleiteten, mit $C_1$–$C_4$-Alkyl und/oder Chlor substituierten Verbindungen, wie N-Hydroxy-t-butylmaleinsäureimid, N-Hydroxy-3,4,5,6-tetrachlorophthalimid und dergleichen. Vorzugsweise steht A–B in obiger Formel I für CH=CH, 1,2-Phenylen oder eine davon abgeleitete, mit $C_1$–$C_4$-Alkyl und/oder Chlor substituierte Gruppe. Besonders bevorzugter Katalysator ist N-Hydroxyphthalimid.

Gemäß dem erfindungsgemäßen Verfahren können Isoprenoide oxidiert werden, welche ein allylisches Wasserstoffatom aufweisen, d.h. Isoprenoide, welche eine Methyl-, Methylen- oder Methingruppe an einer C–C-Doppelbindung besitzen.

Unter dem Begriff «Isoprenoid» werden in der Fachliteratur Verbindungen zusammengefaßt, die in mehr oder weniger offensichtlicher Weise durch Zusammenlagerung von Isopren-Einheiten entstanden sein könnten. Der Ausdruck Isoprenoid umfaßt somit Untergruppen wie Hemiterpene, Terpene, Sesquiterpene, Diterpene, Sesterterpene, Triterpene, Tetraterpene, Carotinoide, Steroide und dergleichen. Die Terpene, Sesquiterpene, Diterpene, Sesterterpene, Triterpene und Tetraterpene sowie ähnliche Verbindungen mit isoprenoidem Bauprinzip werden auch unter dem Begriff Terpenoide zusammengefaßt. Bezüglich der Definition der Ausdrücke Carotinoid, Isoprenoid, Steroid, Terpenoid usw. sei auf Römpps Chemie Lexikon, insbesondere Band 1, 609 (1979), Band 3,

1965 (1983), Band 5, 3336 (1975) und Band 6, 3504 (1977) verwiesen.

Die obigen Begriffe Isoprenoid, Steroid, Terpenoid, Terpen, Sesquiterpen usw. umfassen sowohl Kohlenwasserstoffe mit isoprenoidem Bauprinzip als auch davon abgeleitete Alkohole, Aldehyde, Ketone und Ester. Bevorzugte Ausgangsmaterialien in dem erfindungsgemässen Verfahren sind die isoprenoiden Kohlenwasserstoffe und die davon abgeleiteten Alkohole oder veresterten Alkohole, d.h. Verbindungen mit einer Gruppe RO-, worin R Wasserstoff oder Acyl bezeichnet.

Der Ausdruck «Acyl» bedeutet im Rahmen der vorliegenden Erfindung übliche Acylreste, insbesondere Alkanoylreste und Aroylreste, wie Formyl, Acetyl, Propionyl, Butyryl, Benzoyl und dergleichen. Acylreste mit höchstens 11 Kohlenstoffatomen sind bevorzugt. Besonders bevorzugt sind Acylreste mit höchstens 7 Kohlenwasserstoffatomen, insbesondere Acetyl.

Bevorzugte Ausgangsmaterialien in dem erfindungsgemäßen Verfahren sind die Steroide, Terpene und Sesquiterpene, welche mindestens ein allylisches Wasserstoffatom aufweisen.

Bevorzugte Beispiele derartiger Verbindungen sind Cholesterin (5-Cholesten-3β-ol) und Cholesterinester (3β-Acyloxy-5-cholesten), α-Pinen, β-Pinen, Limonen, Citronellol und Citronellylester, Linalool und Linalylester, Dehydrolinalool und Dehydrolinalylester, α-Terpinen, α-Cedren, Valencen und Isolongifolen.

Die erfindungsgemäße Oxidation erfolgt radikalisch und wird daher wesentlich durch die Stabilität der intermediär gebildeten Radikale beeinflußt. Hierbei können unter Umständen Allylumlagerungen auftreten. Der Reaktionsverlauf und die Art der gebildeten Produkte wird jedoch nach den dem Fachmann bekannten Regeln über die Stabilität von Radikalen bestimmt.

Als Lösungsmittel für die erfindungsgemäße Oxidation wird zweckmäßig ein inerter Ester oder ein inertes Keton verwendet. Gewünschtenfalls können auch Gemische dieser Lösungsmittel untereinander oder mit anderen inerten organischen Lösungsmitteln verwendet werden. Bevorzugte Ester und Ketone sind diejenigen mit höchstens 8 Kohlenstoffatomen und insbesondere diejenigen mit höchstens 6 Kohlenstoffatomen. Eine bevorzugte Gruppe von Estern sind die Alkylalkanoate, insbesondere die Alkylacetate, wie Methylacetat, Äthylacetat, n-Propylacetat, Isopropylacetat, n-Butylacetat, Isobutylacetat und t-Butylacetat. Bevorzugte Ketone sind die Alkanone und Cycloalkanone, wie Aceton, Methyläthylketon, Methylisobutylketon und Cyclohexanon. Besonders bevorzugte Lösungsmittel sind Äthylacetat, Aceton und insbesondere Methylisobutylketon und Cyclohexanon.

Verbindungen mit basischen Gruppen oder «acidem» Wasserstoff können zu ionischen Reaktionen am Katalysator und daher zu teilweiser oder vollständiger Inaktivierung des Katalysators führen. Das Reaktionsgemisch sollte daher mit Vorteil keine basischen Verbindungen mit einem $pK_b$-Wert unterhalb etwa 9, vorzugsweise keine mit einem $pK_b$-Wert unterhalb etwa 11 enthalten. Ferner sollte das Reaktionsgemisch mit Vorteil keine Verbindungen mit «acidem» Wasserstoff enthalten, deren $pK_a$-Wert unterhalb etwa 13 liegt, vorzugsweise keine Verbindungen, deren $pK_a$-Wert unterhalb etwa 15 liegt. Die obigen $pK_a$- und $pK_b$-Werte beziehen sich auf wässrige Lösungen der betreffenden Verbindungen bei 25 °C. Die erfindungsgemäß eingesetzten Ausgangsmaterialien, Katalysatoren und Lösungsmittel weisen keine störenden, sauren oder basischen Gruppen auf. Beispielsweise können nach dem erfindungsgemäßen Verfahren auch Alkohole umgesetzt werden. Es ist jedoch im allgemeinen bevorzugt, Alkohole in veresterter Form einzusetzen.

Die übrigen Reaktionsbedingungen, wie Temperatur, Druck, Sauerstoffkonzentration des verwendeten Gases, Eduktkonzentration und Katalysatormenge, sind nicht kritisch.

Die erfindungsgemäße Oxidation wird im allgemeinen bevorzugt bei Atmosphärendruck und einer Temperatur von etwa 10–125 °C, besonders bevorzugt bei etwa 20–80 °C durchgeführt.

Der Sauerstoffgehalt des verwendeten Gases kann in breiten Grenzen variieren und beträgt im allgemeinen etwa 10–100 Vol.-%, vorzugsweise etwa 20–50 Vol.-%. Zweckmäßigerweise wird als Oxidationsmittel reiner Sauerstoff oder ein Gemisch von Sauerstoff und Inertgas (z.B. Stickstoff, Argon) verwendet. Besonders bevorzugt ist die Verwendung von Luft oder mit Sauerstoff angereicherter Luft.

Die optimale Eduktkonzentration ist abhängig vom eingesetzten Edukt, vom Lösungsmittel, von der Temperatur und dergleichen, liegt aber im allgemeinen in einem Bereich von etwa 0,005–0,3 Mol/l.

Der Katalysator wird im allgemeinen in einer Menge von etwa 0,1–1 Moläquivalent, vorzugsweise etwa 0,25–1 Moläquivalent, bezogen auf die Eduktmenge eingesetzt. Höhere Katalysatormengen sind jedoch nicht störend. Niedrigere Katalysatormengen sind ebenfalls möglich, können aber zu einer Verlangsamung der Reaktion führen.

Die Reaktionszeit kann je nach Bedingungen variieren. Gewünschtenfalls kann der Reaktionsablauf beschleunigt werden durch a) Erwärmen des Reaktionsgemisches, beispielsweise auf eine Temperatur von etwa 50–100 °C, b) Zusatz einer geringen Menge eines Radikalbildners und Erwärmen auf dessen Zerfallstemperatur oder eine höhere Temperatur, c) Bestrahlung mit ultraviolettem Licht, beispielsweise bei etwa 10–40 °C, oder d) Erhöhung der Katalysatormenge. Besonders bevorzugt ist der Zusatz eines Radikalstarters und Erwärmen auf mindestens dessen Zerfallstemperatur. Hierbei können übliche Radikalstarter, wie z.B. Dibenzoylperoxid, verwendet werden.

Nach beendeter Oxidation kann der Katalysator in einfacher Weise, z.B. mit Hilfe eines unpolaren Lösungsmittels, aus dem Reaktionsgemisch abgetrennt und wiederverwendet werden. Beispielsweise kann das Reaktionsgemisch eingeengt, dann mit unpolarem Lösungsmittel versetzt und der Katalysator auskristallisiert werden. Vorzugsweise wird jedoch das bei der Oxidation verwendete Lösungsmittel vollständig abgedampft und der Rückstand in einem unpolaren Lösungsmittel aufgenommen, wobei der Katalysator als unlöslicher Rückstand zurückbleibt. Beispiele geeigneter unpolarer Lösungsmittel sind Kohlenwasserstoffe und unpolare chlorierte Kohlenwasserstoffe, wie Hexan, Tetrachlormethan und dergleichen.

Die erfindungsgemäße, allylische Oxidation von Isoprenoiden führt im allgemeinen zu Hydroperoxiden. Bei der Oxidation von Verbindungen, welche formal einem Dihydroderivat einer aromatischen Verbindung entsprechen, beispielsweise bei Verbindungen mit einem Cyclohexa-1,3-dien-Ring, werden jedoch im allgemeinen die entsprechenden aromatischen Verbindungen erhalten, d.h. die erfindungsgemäße Oxidation entspricht in diesem Fall zumindest formal einer Dehydrierung.

Die erhaltenen Oxidationsprodukte können nach bekannten Methoden aus dem Reaktionsgemisch abgetrennt werden. Erhaltene Hydroperoxide können gewünschtenfalls zu Alkoholen oder Carbonylverbindungen weiter umgesetzt werden. Eine Reinigung der Hydroperoxide ist in diesen Fällen nicht erforderlich.

Die Reduktion der Hydroperoxide zu Alkoholen kann nach bekannten oder an sich bekannten Methoden erfolgen, z.B. mit Alkalimetallsulfit in Wasser, mit Alkalimetalljodid in Eisessig, mit Natriumborhydrid in einem protischen Lösungsmittel, mit Triphenylphosphin in einem aromatischen Lösungsmittel oder durch katalytische Hydrierung.

Primäre und sekundäre Hydroperoxide können durch Wasserabspaltung in die entsprechenden Carbonylverbindungen (Aldehyde oder Ketone) übergeführt werden. Geeignete Dehydratisierungsmittel sind dem Fachmann grundsätzlich bekannt. Vorzugsweise wird die Dehydratisierung jedoch mit Kupfer-(I)-chlorid, Kupfer-(II)-chlorid oder einem Carbonsäureanhydrid, wie Acetanhydrid, Phthalsäureanhydrid und dergleichen, in Pyridin durchgeführt. Bei Verwendung eines Carbonsäureanhydrids werden Verbindungen, welche eine Hydroxygruppe aufweisen, gleichzeitig verestert. Gewünschtenfalls kann die Estergruppe anschliessend nach bekannten Methoden (z.B. durch Verseifen mit Natronlauge) wieder abgespalten werden.

Beispiele besonders bevorzugter Umsetzungen nach dem erfindungsgemäßen Verfahren sind die folgenden Reaktionen:

a) Oxidation von Cholesterin oder Cholesterinestern zum 7-Hydroperoxy-Derivat und, gewünschtenfalls, Dehydratisierung des Hydroperoxids zu 7-Ketocholesterin oder 7-Ketocholesterinestern

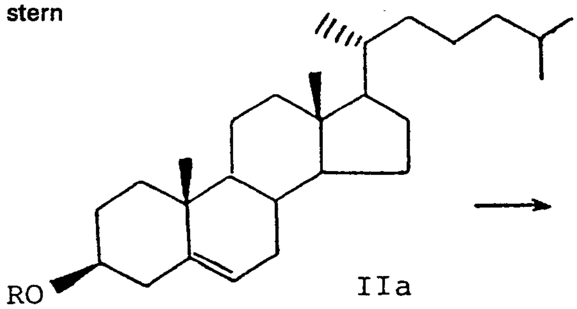

IIa

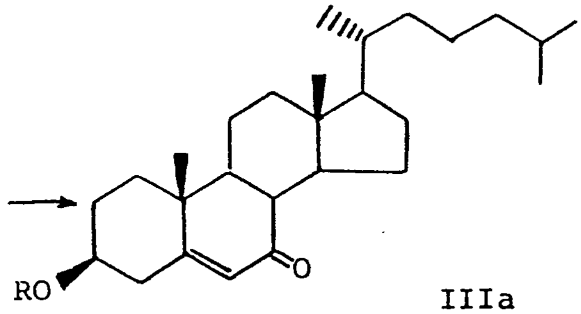

IIIa

b) Oxidation von α-Pinen zum Hydroperoxy-Derivat und, gewünschtenfalls, Dehydratisierung des Hydroperoxids zu Verbenon

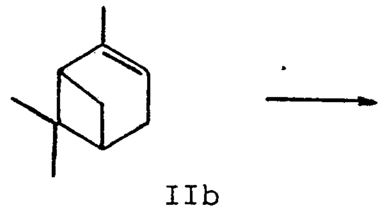

IIb

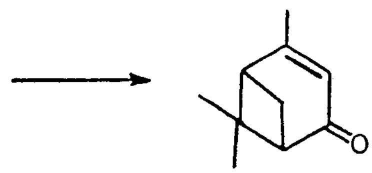

IIIb

c) Oxidation von Citronellol oder Citronellylestern und, gewünschtenfalls, Reduktion des erhaltenen Hydroperoxids zum 3,7-Dimethyl-5-octen-1,7-diol oder dessen Halbester

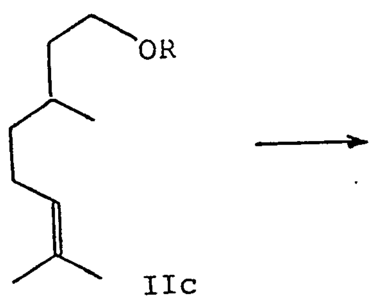

IIc

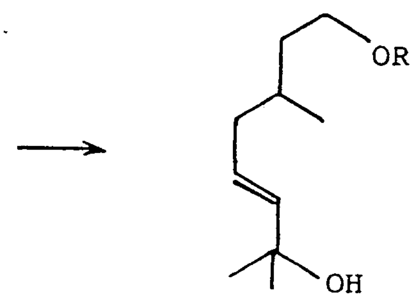

IIIc

d) Oxidation von Linalool, Dehydrolinalool oder deren Ester und, gewünschtenfalls, Reduktion des erhaltenen Hydroperoxids zu 2,6-Dimethyl-3,7-octadien-2,6-diol, 2,6-Dimethyl-3-octen-7-in-2,6-diol oder deren Halbester.

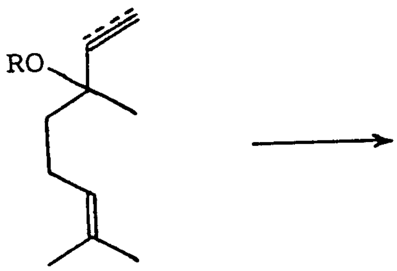

IId

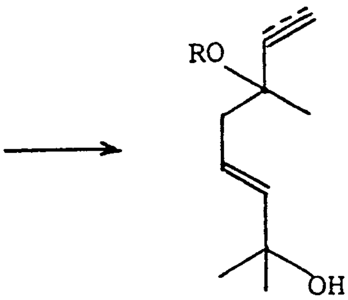

IIId

e) Oxidation (Dehydrierung) von α-Terpinen zu p-Cymol

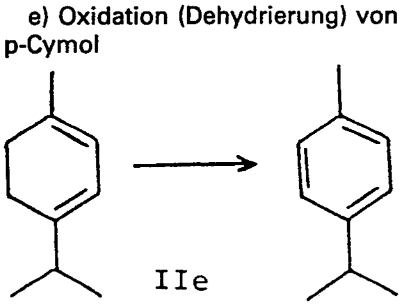

IIe IIIe

f) Oxidation von α-Cedren und, gewünschtenfalls, Dehydratisierung des erhaltenen Hydroperoxids zu Cedrenon

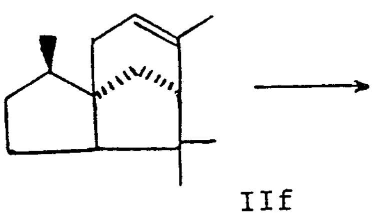

IIf

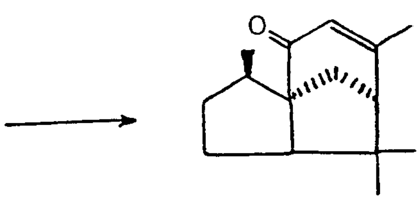

IIIf

g) Oxidation von Valencen und, gewünschtenfalls, Dehydratisierung des erhaltenen Hydroperoxids zu Nootkaton

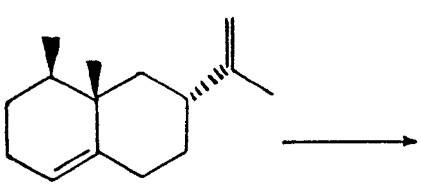

IIg

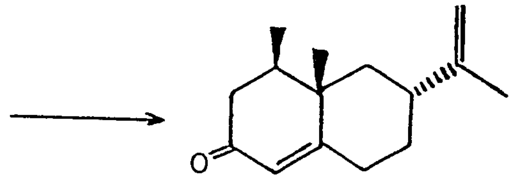

IIIg

h) Oxidation von Isolongifolen und, gewünschtenfalls, Dehydratisierung des erhaltenen Hydroperoxids zu Isolongifolenon

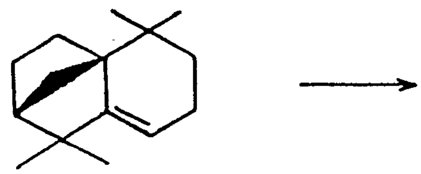

IIh

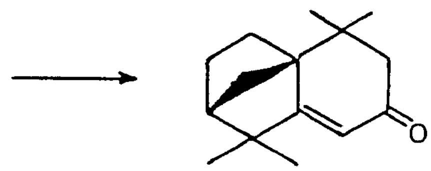

IIIh

In den Formeln IIa, IIIa, IIc, IIIc, IId und IIId bedeutet R Wasserstoff oder Acyl, vorzugsweise eine der oben erwähnten Acylgruppen. In den Formeln IId und IIId bezeichnet das Symbol ---- gegebenenfalls eine zusätzliche C–C-Bindung. In den Formeln IIa, IIIa, IIf, IIIf, IIg, IIIg, IIh und IIIh bedeutet das Symbol ▾, daß die betreffende Bindung über der Zeichenebene liegt, und das Symbol ≡, daß die betreffende Bindung unter der Zeichenebene liegt. Im Falle von Formeln IIg und IIIg bedeutet letzteres Symbol, daß der Isopropenylrest unter der Zeichenebene liegt.

Das erfindungsgemäße Verfahren wird durch die folgenden Beispiele weiter veranschaulicht.

Beispiel 1

In einem Sulfierkolben, versehen mit Begasungsrührer, Thermometer und Rückflußkühler wurde eine Lösung von 16,3 g (0,1 Mol) N-Hydroxyphthalimid in 350 ml Aceton zum Sieden erhitzt und nacheinander mit 42,8 g (0,1 Mol) Cholesterinacetat und 0,16 g (0,5 mMol) Dibenzoylperoxid versetzt. Anschließend wurde unter Rühren bei Rückflußtemperatur während 9 h ein schwacher Preßluftstrom in die Reaktionslösung eingeleitet. Danach wurde das auf Raumtemperatur abgekühlte Reaktionsgemisch am Rotationsverdampfer (Badtemperatur 40–50 °C) zur Trockene eingedampft. Der Rückstand wurde mit 200 ml Tetrachlormethan versetzt und auf 40 °C erwärmt. Der erhaltene feinkristalline Niederschlag wurde nach Abkühlung des Reaktionsgemisches auf Raumtemperatur abgenutscht, mit 50 ml Tetrachlormethan gewaschen und im Wasserstrahlvakuum bei 80 °C 4 h getrocknet. Hierbei wurden 16,0 g (98,2%) N-Hydroxyphthalimid zurückgewonnen.

Das Filtrat wurde am Rotationsverdampfer eingedampft und der ölige Rückstand unter Rühren bei 50 °C in 200 ml Pyridin aufgenommen. Diese Lösung wurde auf 5–10 °C abgekühlt und unter Kühlung mit 20 ml Acetanhydrid versetzt. Das Gemisch wurde über Nacht bei Raumtemperatur stehengelassen und dann am Rotationsverdampfer eingeengt. Der Rückstand wurde mit 100 ml Methanol versetzt und bei 50 °C gerührt. Die erhaltene Suspension wurde 1 h in einem Eisbad gekühlt und dann abgenutscht. Die Kristalle wurden auf der Nutsche mit 50 ml Methanol gewaschen und dann im Wasserstrahlvakuum 4 h bei 80 °C getrocknet. Hierbei wurden 32,6 g (73,8%) 7-Ketocholesterinacetat als schwach gelbe Kristalle mit Smp. 151–154 °C erhalten. Dieses Rohprodukt wurde durch Kochen am Rückfluß in 150 ml Aceton gelöst. Die Lösung wurde auf 80 ml eingeengt und 3 h im Eisbad gekühlt. Das erhaltene Kristallisat wurde abgenutscht, auf der Nutsche mit 20 ml Aceton gewaschen und im Wasserstrahlvakuum 4 h bei Raumtemperatur getrocknet. Es resultierten 29,8 g (67,4%) 7-Ketocholesterinacetat in Form farbloser Kristalle mit Smp. 156–159 °C.

Beispiel 2

6,1 g (0,037 Mol) N-Hydroxyphthalimid wurden in 175 ml Isobutylmethylketon bei 54 °C gelöst. Die Lösung wurde mit 16 g (0,037 Mol) Cholesterinacetat und 0,06 g (0,25 mMol) Dibenzoylperoxid versetzt. Anschließend wurde bei 54 °C und unter intensivem Rühren während ca. 9 h Luft (250 ml/min) in die Reaktionslösung eingeleitet. Nach beendeter Oxidation wurde das auf Raumtemperatur abgekühlte Reaktionsgemisch zur Trockene eingedampft. Zur Rückgewinnung des N-Hydroxyphthalimids wurde der Rückstand mit 75 ml Tetrachlormethan versetzt und das Gemisch 1 h bei 40 °C gerührt und dann bei 20 °C filtriert. Auf diese Weise wurden 92% des N-Hydroxyphthalimids zurückgewonnen.

Das Filtrat wurde eingedampft und der Rückstand unter Rühren mit 75 ml Pyridin versetzt. Anschließend wurde das Gemisch bei 5–10 °C mit 7,5 ml Acetanhydrid versetzt, über Nacht bei Raumtemperatur stehengelassen und dann eingedampft. Der Rückstand wurde mit 40 ml Methanol versetzt. Das Gemisch wurde 15 min bei 50 °C und dann 1 h im Eisbad gerührt und anschließend filtriert. Es resultierte kristallines 7-Ketocholesterinacetat (Reinheit 97%) in einer Ausbeute von 72%.

In analogen Versuchen wurde anstelle von 175 ml Isobutylmethylketon als Lösungsmittel 175 ml Äthylmethylketon, 175 ml Diäthylketon oder 175 ml Cyclohexanon eingesetzt. Bei gleichen Reaktionsbedingungen und gleicher Aufarbeitung betrug die Ausbeute an isoliertem 7-Ketocholesterinacetat bei Verwendung von Äthylmethylketon 66%, bei Verwendung von Diäthylketon 68% und bei Verwendung von Cyclohexanon 80%.

Beispiel 3

Nach der in Beispiel 2 beschriebenen Methode wurden die in Tabelle 1 zusammengestellten Versuche durchgeführt. In Versuch (a) wurde Cholesterin als Edukt eingesetzt, in den übrigen Versuchen Cholesterinacetat. Die Überführung des Hydroperoxids in das Keton durch Wasserabspaltung wurde mit Acetanhydrid in Pyridin durchgeführt, mit Ausnahme von Versuch (d), in welchem die Wasserabspaltung mit $CuCl_2 \cdot 2H_2O$ in Pyridin durchgeführt wurde. Da in Versuch (a) bei der Wasserabspaltung mit Acetanhydrid in Pyridin gleichzeitig die Hydroxygruppe verestert wurde, wurde in allen Versuchen 7-Ketocholesterinacetat als Endprodukt erhalten. In Tabelle 1 bedeuten T die Reaktionstemperatur und t die Reaktionszeit für die Oxidation mit Luft. Die angegebenen Ausbeuten an 7-Ketocholesterinacetat beziehen sich auf umgesetztes Edukt. Die Reaktionsbedingungen wurden im allgemeinen nicht optimiert.

Tabelle 1

| Versuch | Katalysator | Aktivator | Lösungsmittel | T | t | Umsatz | Ausbeute |
|---|---|---|---|---|---|---|---|
| (a) | NHPI | DBP | $CH_3COCH_3$ | 52–53 °C | 9h | 100% | 66% |
| (b) | NHPI | – | $CH_3COCH_3$ | 52–53 °C | 24h | 100% | 75,3% |
| (c) | NHPI | – | $CH_3COO(CH_2)_3CH_3$ | 118–124 °C | 2h | 100% | 67,4% |
| (d) | NHPI | hv | $CH_3COCH_3$ | 14 °C | 8h | 100% | 45% |
| (e) | NHPI | DBP | $CH_3COOCH_2CH_3$ | 73–74 °C | 2h | 94,2% | 68,2% |
| (f) | N-Hydroxymaleinsäureimid | DBP | $CH_3COOCH_2CH_3$ | 73–74 °C | 6h | 70,5% | 91,4% |
| (g) | N-Hydroxy-t-butylmaleinsäureimid | DBP | $CH_3COOCH_2CH_3$ | 73–74 °C | 6h | 95,9% | 70,2% |
| (h) | N-Hydroxysuccinimid | DBP | $CH_3COOCH_2CH_3$ | 73–74 °C | 6h | 62,0% | 65,1% |
| (i) | N-Hydroxy-3,4,5,6-tetrachlorophthalimid | DBP | $CH_3COOCH_2CH_3$ | 73–74 °C | 6h | 100% | 61,3% |
| (j) | N-Hydroxy-2,3-naphthalindicarbonsäureimid | DBP | $CH_3COCH_3$ | 52–53 °C | 24h | 95,5% | 53,2% |

NHPI = N-Hydroxyphthalimid
DBP = Dibenzoylperoxid
hv = Bestrahlung mit Quecksilberdampflampe (150 W)

Beispiel 4

In ein Gemisch von 4,1 g (–)-α-Pinen, 4,8 g N-Hydroxyphthalimid, 0,05 g Dibenzoylperoxid (enthaltend 20–25% Wasser) und 105 ml Isobutylmethylketon wurde unter Rühren bei 54–55 °C ein Luftstrom (16 l/h) eingeleitet. Nach 6 h waren 44% des Edukts umgesetzt. Die Oxidation wurde abgebrochen und das Lösungsmittel abgedampft. Der Rückstand wurde in Hexan/Diäthyläther (Vol. 1:1) aufgenommen. Nach Abtrennung des N-Hydroxyphthalimids durch Filtration wurde das Filtrat zur Trockene eingedampft.

Der Rückstand wurde in 25 ml Pyridin aufgenommen und die gekühlte Lösung mit 2,5 ml Acetanhydrid versetzt. Nach 16 h wurde das Reaktionsgemisch in Wasser/Diäthyläther verteilt. Die organische Phase wurde abgetrennt, getrocknet und eingedampft. Das zurückbleibende Rohprodukt (2,0 g) wurde an Kieselgel mit Hexan/Diäthyläther (Vol. 7:3) chromatographiert und dann destilliert. Es resultierten 1,3 g (–)-Verbenon in einer chemischen Reinheit von 77,7%; Ausbeute 51% bezogen auf umgesetztes (–)-α-Pinen.

Beispiel 5

In ein Gemisch von 6,45 g Citronellolacetat (Reinheit 92%), 4,8 g N-Hydroxyphthalimid und 105 ml Aceton wurde unter intensivem Rühren am Rückfluß Luft (250 ml/min) eingeleitet. Nach 6 h waren 81,7% des Citronellolacetats umgesetzt. Die Oxidation wurde abgebrochen und das Lösungsmittel abgedampft. Der Rückstand wurde in Tetrachlormethan/Hexan (Vol. 1:1) aufgenommen. Durch Filtration konnten 4,67 g N-Hydroxyphthalimid zurückgewonnen werden. Das Filtrat wurde eingedampft. Das zurückbleibende Öl wurde an Kieselgel mit Dichlormethan/Aceton (Vol. 95:5) chromatographiert. Hierbei wurden 4,46 g (E)-7-Hydroperoxy-3,7-dimethyl-5-octen-1-ylacetat in einer Reinheit von 95% erhalten; $n_D^{23}$ = 1,460; Ausbeute 75,2%, bezogen auf umgesetztes Citronellolacetat.

Beispiel 6

In ein Gemisch von 10 g Linalolacetat (Reinheit 95%), 8,3 g N-Hydroxyphthalimid, 0,81 g Dibenzoylperoxid und 300 ml Äthylacetat wurde unter intensivem Rühren am Rückfluß während 10 h Luft (250 ml/min) eingeleitet. Danach wurde das Lösungsmittel abgedampft und der Rückstand in 150 ml Tetrachlormethan aufgenommen. Das Gemisch wurde 1,5 h bei Raumtemperatur gerührt und dann filtriert. Hierbei wurden 7,4 g N-Hydroxyphthalimid zurückgewonnen. Das Filtrat wurde eingedampft, wobei 13,83 g gelbliches Öl zurückblieben. 2,0 g des erhaltenen, öligen Rückstandes wurden an Kieselgel mit Cyclohexan/Diäthyläther (Vol. 1:1) chromatographiert. Hierbei wurden 0,90 g 2-Hydroperoxy-2,6-dimethyl-3,7-octadien-6-ylacetat als farbloses Öl erhalten. Die Verbindung wurde mittels Infrarot-, Kernresonanz- und Massenspektroskopie charakterisiert.

Die restlichen 11,83 g des öligen Rückstandes wurden in 250 ml Äthanol gelöst. Die Lösung wurde mit 15,3 g Natriumjodid und 5,85 ml Eisessig versetzt, 3 h bei Raumtemperatur gerührt und dann eingedampft. Der Rückstand wurde in 200 ml Dichlormethan gelöst und die Lösung einmal mit 200 ml und zweimal mit je 125 ml 20%iger Natriumthiosulfat-Lösung gewaschen. Die wäßrigen Phasen wurden zweimal mit je 125 ml Dichlormethan nachextrahiert. Die organischen Phasen wurden über Natriumsulfat getrocknet, filtriert und eingedampft.

Der erhaltene ölige Rückstand (10,49 g) wurde in 100 ml Methanol gelöst. Die Lösung wurde mit 4,5 g Kaliumcarbonat versetzt und 8 h bei 40 °C und anschließend noch 16 h bei Raumtemperatur gerührt. Danach wurde das Reaktionsgemisch eingedampft und der Rückstand mit 150 ml Diäthyläther versetzt. Das Gemisch wurde 30 min bei Raumtemperatur gerührt und filtriert. Der nach dem Eindampfen des Filtrates erhaltene rote ölige Rückstand (6,26 g) wurde an Kieselgel mit Dichlormethan/Äthylacetat (Vol. 1:1) chromatographiert. Hierbei wurden 3,27 g 2,6-Dimethyl-3,7-octadien-2,6-diol in Form eines rötlichen Öles isoliert. ($n_D^{23}$ = 1,474); Ausbeute 37,7%, bezogen auf Linalolacetat. Die restlichen Fraktionen, welche zum Teil ebenfalls noch Produkt enthielten, wurden nicht aufgearbeitet.

Beispiel 7

Nach der in den Beispielen 1–6 beschriebenen Methode wurden die in Tabelle 2 genannten Edukte in Gegenwart von N-Hydroxyphthalimid mit Luft oxidiert. Alle Versuche wurden in Aceton bei Rückflußtemperatur des Reaktionsgemisches und unter Zusatz von Dibenzoylperoxid durchgeführt.

Versuch (e) ergab p-Cymol als Oxidationsprodukt. In den Versuchen (a)–(d) wurden als Oxidationsprodukte Hydroperoxide erhalten, welche in analoger Weise zu den Beispielen 1–4 mit Acetanhydrid in Pyridin zu den in Tabelle 2 genannten Ketonen weiter umgesetzt wurden.

In Tabelle 2 bedeutet t die Reaktionszeit für die Oxidation mit Luft. Die angegebenen Ausbeuten an Produkt sind bezogen auf umgesetztes Edukt. Die Reaktionsbedingungen wurden nicht optimiert.

Tabelle 2

| Versuch | Edukt | t | Umsatz | Ausbeute | Produkt |
|---|---|---|---|---|---|
| (a) | α-Pinen | 13h | 92% | 36,3% | Verbenon |
| (b) | Valencen | 24h | 94,8% | 55,6% | Nootkaton |
| (c) | Isolongifolen | 35h | 100% | 68% | Isolongifolenon |
| (d) | α-Cedren | 16,5h | 100% | 46,1% | Cedrenon |
| (e) | α-Terpinen | 8,5h | 100% | 74% | p-Cymol |

**Patentansprüche**

1. Verfahren zur katalytischen Oxidation allylischer Gruppen mit Sauerstoff oder einem sauerstoffhaltigen Gas, dadurch gekennzeichnet, daß man ein Isoprenoid, welches mindestens ein allylisches Wasserstoffatom aufweist, in einem inerten Keton oder Ester in Gegenwart eines N-Hydroxydicarbonsäureimids der allgemeinen Formel

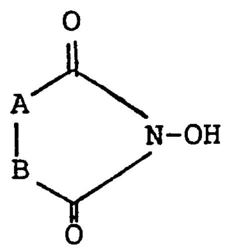
I

worin A–B für $CH_2$-$CH_2$, CH=CH, einen aromatischen Kohlenwasserstoffrest oder eine von diesen Gruppen abgeleitete Gruppe steht, worin ein oder mehrere Wasserstoffatome durch Alkyl oder Halogen ersetzt sind, oxidiert und daß man, gewünschtenfalls, ein erhaltenes Hydroperoxid zum Alkohol reduziert oder ein erhaltenes primäres oder sekundäres Hydroperoxid zur Carbonylverbindung dehydratisiert.

2. Verfahren nach Anspruch 1, dadurch gekennzeichnet, daß man Cholesterin oder einen Cholesterinester umsetzt.

3. Verfahren nach Anspruch 1, dadurch gekennzeichnet, daß man ein Terpen oder Sesquiterpen umsetzt.

4. Verfahren nach Anspruch 3, dadurch gekennzeichnet, daß man α-Pinen, Valencen, α-Cedren oder Isolongifolen umsetzt.

5. Verfahren nach einem der Ansprüche 1 bis 4, dadurch gekennzeichnet, daß man die Oxidation in Gegenwart einer Verbindung der Formel I durchführt, worin A–B für CH=CH, 1,2-Phenylen oder eine von diesen Gruppen abgeleitete, mit $C_1$–$C_4$-Alkyl oder Chlor substituierte Gruppe steht.

6. Verfahren nach Anspruch 5, dadurch gekennzeichnet, daß man die Oxidation in Gegenwart von N-Hydroxyphthalimid durchführt.

7. Verfahren nach einem der Ansprüche 1 bis 6, dadurch gekennzeichnet, daß man die Oxidation in einem Alkanon, Cycloalkanon oder Alkylalkanoat mit höchstens 8 Kohlenstoffatomen durchführt.

8. Verfahren nach Anspruch 7, dadurch gekennzeichnet, daß man die Oxidation in Methylisobutylketon, Aceton, Cyclohexanon oder Äthylacetat durchführt.

9. Verfahren nach einem der Ansprüche 1 bis 8, dadurch gekennzeichnet, daß man als Oxidationsmittel Luft oder mit Sauerstoff angereicherte Luft verwendet.

10. Verfahren nach einem der Ansprüche 1 bis 9, dadurch gekennzeichnet, daß man die Oxidation unter Zusatz eines Radikalstarters und Erwärmen des Reaktionsgemisches auf mindestens die Zerfallstemperatur des Radikalstarters durchführt.

**Claims**

1. A process for the catalytic oxidation of allylic groups with oxygen or an oxygen-containing gas, characterized by oxidizing an isoprenoid, which has at least one allylic hydrogen atom, in an inert ketone or ester in the presence of a N-hydroxydicarboxylic acid imide of the general formula

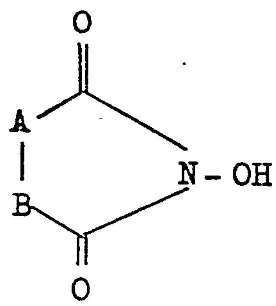
I

wherein A–B stands for $CH_2$-$CH_2$, CH=CH, an aromatic hydrocarbon residue or a group derived from one of these groups in which one or more hydrogen atoms is/are replaced by alkyl or halogen, and, if desired, reducing a hydroperoxide obtained to the alcohol or dehydrating a primary or secondary hydroperoxide obtained to the carbonyl compound.

2. A process according to claim 1, characterized in that cholesterol or a cholesterol ester is reacted.

3. A process according to claim 1, characterized in that a terpene or sesquiterpene is reacted.

4. A process according to claim 3, characterized in that α-pinene, valencene, α-cedrene or isolongifolene is reacted.

5. A process according to any one of claims 1 to 4, characterized in that the oxidation is carried out in the presence of a compound of formula I in which A–B stands for CH=CH, 1,2-phenylene or a group which is derived from one of these groups and which is substituted with $C_1$–$C_4$-alkyl or chlorine.

6. A process according to claim 5, characterized in that the oxidation is carried in the presence of N-hydroxyphthalimide.

7. A process according to any one of claims 1 to 6, characterized in that the oxidation is carried out in an alkanone, cycloalkanone or alkyl alkanoate with a maximum of 8 carbon atoms.

8. A process according to claim 7, characterized in that the oxidation is carried out in methyl isobutyl ketone, acetone, cyclohexanone or ethyl acetate.

9. A process according to any one of claims 1 to 8, characterized in that air or air enriched with oxygen is used as the oxidation agent.

10. A process according to any one of claims 1 to 9, characterized in that the oxidation is carried out with the addition of a radical initiator and heating the reaction mixture to at least the decomposition temperature of the radical initiator.

**Revendications**

1. Procédé pour l'oxydation catalytique de groupes allyliques à l'aide d'oxygène ou d'un gaz contenant de l'oxygène, caractérisé en ce que l'on oxyde un isoprénoïde contenant au moins un atome d'hydrogène allylique, dans une cétone inerte ou un ester inerte, en présence d'un N-hydroxydicarboximide de formule générale

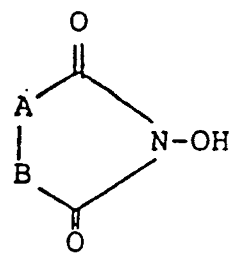

I

dans laquelle A–B représente $CH_2$-$CH_2$, CH=CH, un groupe hydrocarboné aromatique ou un groupe dérivé de ces derniers dans lequel un ou plusieurs atomes d'hydrogène sont remplacés par des groupes alkyle ou des halogènes, et en ce que, si on le désire, on convertit un hydroperoxyde ainsi obtenu en l'alcool par réduction, ou un hydroperoxyde primaire ou secondaire ainsi obtenu en le composé carbonylé par déshydratation.

2. Procédé selon la revendication 1, caractérisé en ce que l'on convertit le cholestérol ou un ester du cholestérol.

3. Procédé selon la revendication 1, caractérisé en ce que l'on convertit un terpène ou un sesquiterpène.

4. Procédé selon la revendication 3, caractérisé en ce que l'on convertit l'alpha-pinène, le valencène, l'alpha-cédrène ou l'isolongifolène.

5. Prodécé selon l'une des revendications 1 à 4, caractérisé en ce que l'on effectue l'oxydation en présence d'un composé de formule I dans laquelle A–B représente CH=CH, un groupe 1,2-phénylène ou un groupe dérivé de ces derniers et substitué par des groupes alkyle en $C_1$–$C_4$ ou le chlore.

6. Procédé selon la revendication 5, caractérisé en ce que l'on effectue l'oxydation en présence du N-hydroxyphthalimide.

7. Procédé selon l'une des revendications 1 à 6, caractérisé en ce que l'on effectue l'oxydation dans une alcanone, une cycloalcanone ou un alcanoate d'alkyle à 8 atomes de carbone au maximum.

8. Procédé selon la revendication 7, caractérisé en ce que l'on effectue l'oxydation dans la méthylisobutylcétone, l'acétone, la cyclohexanone ou l'acétate d'éthyle.

9. Procédé selon l'une des revendications 1 à 8, caractérisé en ce que l'on utilise en tant qu'agent oxydant l'air ou de l'air enrichi en oxygène.

10. Procédé selon l'une des revendications 1 à 9, caractérisé en ce que l'on effectue l'oxydation en présence d'un inducteur radicalaire et en chauffant le mélange de réaction à une température au moins égale à la température de décomposition de l'inducteur radicalaire.